# EUROPEAN PATENT SPECIFICATION

(11) **EP 2 772 759 B1**
(45) Date of publication and mention of the grant of the patent: **12.10.2016**
(21) Application number: 13156980.8
(22) Date of filing: 27.02.2013
(51) Int. Cl.: G01N 33/574

(54) **Composition for diagnosis of lung cancer**
Zusammensetzung für die Diagnose von Lungenkrebs
Composition pour diagnostic du cancer des poumons

(43) Date of publication of application: 03.09.2014
(73) Proprietor: Seoul National University R&DB Foundation, Seoul 151-742 (KR)
(72) Inventor: Cho, Je Yoel, Seoul 135-506 (KR); Ahn, Jung Mo, Daegu 706-951 (KR)
(74) Representative: Peter, Julian

(56) References cited:
- WO-A2-02/074230
- WO-A2-2011/150994
- GOSWAMI B ET AL: "Paraoxonase: A multifaceted biomolecule", CLINICA CHIMICA ACTA, ELSEVIER BV, AMSTERDAM, NL, vol. 410, no. 1-2, 8 December 2009 (2009-12-08), pages 1-12, XP026738579, ISSN: 0009-8981, DOI: 10.1016/J.CCA.2009.09.025 [retrieved on 2009-09-30]
- SAMRA Z Q ET AL: "Determination of oxygen derived free radicals producer (xanthine oxidase) and scavenger (paraoxonasel) enzymes and lipid parameters in different cancer patients", CLINICAL LABORATORY 2011 VERLAG KLINISCHES LABOR GMBH DEU, vol. 57, no. 9-10, 2011, pages 741-747, XP9170711, ISSN: 1433-6510

## Description

### 1. Field of the Invention

The present invention relates to a method for diagnosis of small cell lung cancer from biological sample.

### 2. Description of the Related Art

In spite of great advances in modern medicine, cancer incidence and cancer-derived death are still increasing. For this reason, individual, national and social losses due to diagnosis and treatment of lung cancer are also continuously increasing. In order to minimize cancer incidence and cancer-induced death through integrated cancer management and reduce the expense burden, an early medical examination of cancer is required and, in terms of a screening concept, there is now a strong need to develop a biomarker capable of easily and rapidly making a diagnosis of cancer for a large number of people and ultimately increasing a survival rate of cancer patients.

Lung cancer is known to rank first in mortality and second or third in cancer incidence at home and abroad, and is generally diagnosed in a progressed state of at least Stage II at the time of diagnosis, and therefore, shows a very low survival rate. Accordingly, although early diagnosis is important, a biomarker for early diagnosis of lung cancer using blood has yet to be developed.

Lung cancer is classified into small cell lung cancer (SCLC) and non-small cell lung cancer (NSCLC) in terms of the size of cells. SCLC holds about 20% of overall lung cancers, comprises tumor cells derived from neuroendocrine and shows very rapid metastasis, and the prognosis of an SCLC patient is relatively poor. Most SCLC patients are already in the terminal stage at the time of diagnosis and show a five year survival rate of only 5 to 10%.

Current diagnosis of lung cancer includes chest X-ray photography, chest computerized topography (CT), magnetic resonance imaging (MRI), bronchial endoscopy, etc., however, in the case of the chest CT, lung cancer cannot be found unless it has a size of about 0.1 cm or more. Alternatively, although a method using a bronchial endoscope directly monitors inside the lung by inserting the endoscope through the bronchial tube, it is difficult to observe a tumor at the end of the lung.

Recently, proteomics techniques and biomarkers have been usefully adapted for diagnosis of lung cancer. Proteomics enables global screening in samples of complicated proteins, and can provide quantitative evidence of varied protein expression and allow studies for direct identification of sites where post-translational modification occurs. Also, studies of proteomics may provide information for titer change by alternative splicing, post-translational modification, and protein interaction. Since the beginning of the post-genomics age, proteins capable of better expressing various and dynamic changes in disease conditions have been studied in about 25,000 gene research projects, and therefore, the above technique has been actively used and developed as a method of discovering biomarkers.

In recent years, studies using 2-DE bonded to MALDI-TDF or 2-DE bonded to LC-MS/MS, and high-throughout western blot to identify a specific protein, which is differently expressed in blood serums of a lung cancer patient and a normal person, have been proceeded.

Among current lung cancer diagnostic markers, neuron-specific enolase (NSE), a carcinoembryonic antigen (CEA) and a cytokeratin 19-fragment neuron (CYFRA21-1) are known as a serum marker. However, these materials do not have sufficient sensitivity and specificity for lung cancer and, in particular, a biomarker useful for diagnosis of SCLC has not yet been disclosed. Accordingly, it is strongly necessary to develop a biomarker enabling simple and early diagnosis of SCLC from blood to thus increase the survival of patients.

### [SUMMARY OF THE INVENTION]

The present invention is directed to discovery of a lung cancer-specific biomarker and a quick, simple and precise diagnosis of lung cancer. In particular, the present invention is directed to finding a biomarker specific to SCLC. There is provided a composition for diagnosis (often referred to as a 'diagnostic composition') of lung cancer, including an antibody that specifically binds to serum paraoxonase 1.

According to the present invention, the above protein is characterized by fucosylation and shows decreased expression in the serum of an SCLC patient, compared to the serum of a normal person.

According to the present invention, the serum paraoxonase 1 protein described above shows decreased expression in the serum of an SCLC patient, compared to the serum of a normal person. However, as compared to the serum of the normal person, fucosylation is increased in the serum of a patient in the terminal stage of SCLC ('a terminal SCLC patient').

There is provided a diagrosis kit for SCLC, including the above composition as an effective ingredient.

There is provided a micro-array for diagnosis of SCLC, there is provided a diagnostic composition for lung cancer, including a primer or probe specific to a nucleic acid encoding serum paraoxonase 1

According to the present invention, the above protein is characterized by fucosylation and shows decreased expression in the serum of an SCLC patient, compared to the serum of a normal person.

According to the present invention, the serum paraoxonase 1 protein is characterized by decreased expression in the serum of an SCLC patient, compared to the serum of a normal person. However, fucosylation is increased in the serum of the SCLC patient, compared to the serum of the normal person.
there is provided a diagnosis kit for SCLC, including the above composition as an effective ingredient.
there is provided a micro-array for diagnosis of SCLC, including the above composition as an effective ingredient.

According to another aspect of the present invention, there is provided a method of detecting serum paraoxonase 1, as a lung cancer marker, from a biological sample, through antigen-antibody binding reaction that uses an antibody that specifically binds to at least one protein selected from the above group.

According to the present invention, the biological sample may include at least one selected from a group consisting of tissue, cell, whole blood, serum, plasma, saliva, sputum, and urine, which are obtained from mammals.

There is provided a diagnostic composition for lung cancer, which has excellent sensitivity and specificity useful for early diagnosis of lung cancer.

### [BRIEF DESCRIPTION OF THE DRAWINGS]

The above and other objects, features and other advantages of the present invention will be more clearly conjunction with the accompanying drawings, in which:

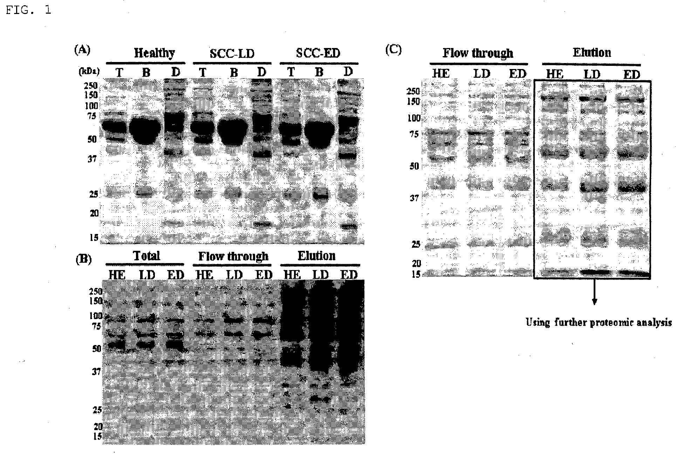

FIG. 1 illustrates (A) SDS PAGE gel electrophoresis results; (B) AAL lectin blot results of fucosylated glycoprotein; and (C) Coomassie staining results for mass spectroscopy of three serum sample groups (i.e., serums of normal persons (Healthy, HE), serums of early SCLC patients (SCC-LD, LD), and serums of terminal SCLC patients (SCC-ED, ED)) according to the present invention, wherein albumin and IgG are depleted from each of their serums.

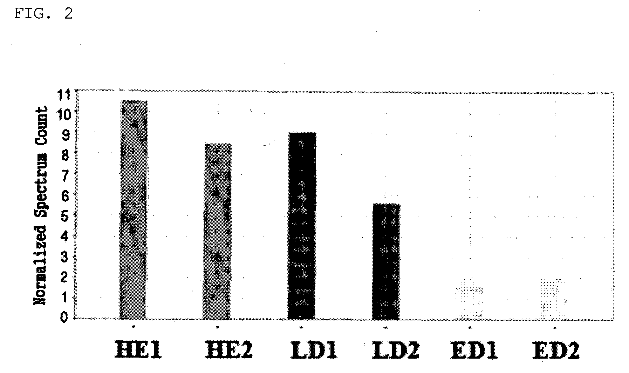

FIG. 2 illustrates mass spectroscopic results of serum paraoxonase 1 protein by comparing and assaying concentrated and fucosylated proteins in three serum sample groups (i.e., serums of normal persons (Healthy, HE), serums of early SCLC patients (SCC-LD, LD) and serums of terminal SCLC patients (SCC-ED, ED)) according to the present invention.

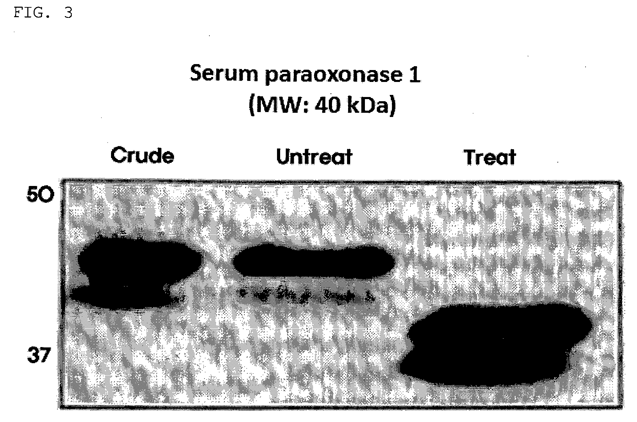

FIG. 3 illustrates a result of identifying whether a sugar chain containing fucose is bonded to serum paraoxonase 1 by treating the serum paraoxonase 1 specific to SCLC with a sugar chain removing enzyme, i.e., PNGase F.

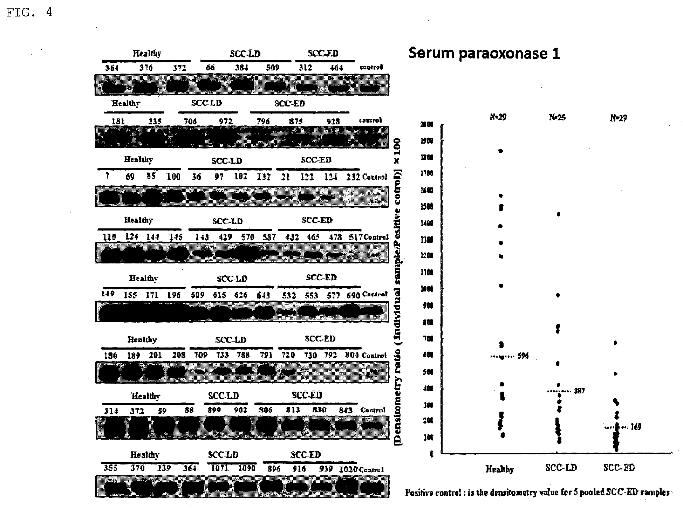

FIG. 4 illustrates (A) results of observing expression levels of serum paraoxonase 1 proteins obtained from three serum sample groups (i.e., serums of normal persons (Healthy, 29 persons), serums of early SCLC patients (SCC-LD, 25 persons) and serums of terminal SCLC patients (SCC-ED, 29 persons)), respectively, through western blot, and (B) results persons)), respectively, through western blot, and (B) results of densitometry measurement to quantitatively exhibit the results of (A).

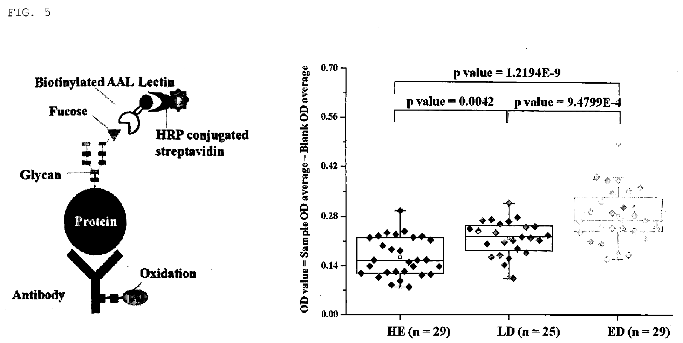

FIG. 5 illustrates results of measuring fucosylation levels of serum paraoxonase 1 proteins obtained from three serum sample groups (i.e., serums of normal persons (Healthy, HE), serums of early SCLC patients (SCC-LD, LD) and serums of terminal SCLC patients (SCC-ED, ED)), respectively, by a hybrid ELISA method.

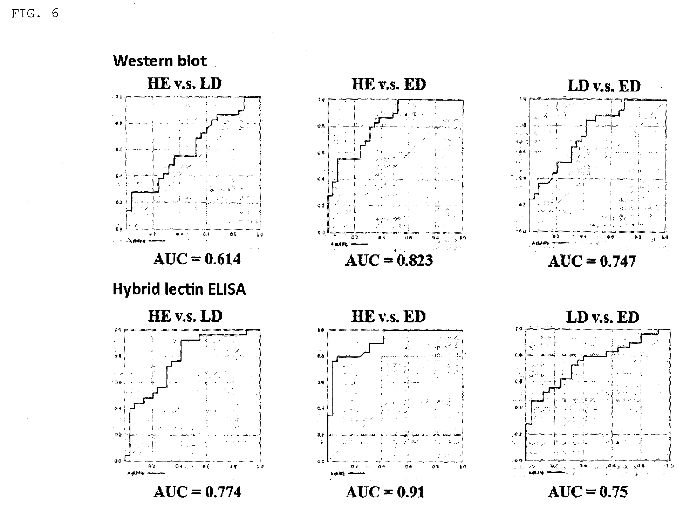

FIG. 6 illustrates levels of serum paraoxonase 1 proteins through western blot and fucosylation levels of serum paraoxonase 1 by hybrid lectin ELISA through ROC curve, with regard to three serum sample groups (i.e., serums of normal persons (Healthy, HE), serums of early SCLC patients (SCC-LD, LD) and serums of terminal SCLC patients (SCC-ED, ED)), respectively.

### [DETAILED DESCRIPTION OF EMBODIMENTS]

Hereinafter, the present invention will be described in detail with reference to the accompanying drawings.

Here is provided a diagnostic composition for lung cancer, including an antibody that specifically binds to at least one protein selected from a group consisting of serum paraoxonase 1, Preferably, the above lung cancer diagnosis composition may include an antibody that specifically binds to the marker, as an effective ingredient.

According to the present invention, the protein is characterized by fucosylation and shows decreased expression in the serum of an SCLC patient, compared to the serum of a normal person.

According to the present invention, the serum paraoxonase 1 protein shows decreased expression in the serum of a terminal SCLC patient (SCC-ED), compared to the serum of a normal person (Healthy, HE). However, as compared to HE, the fucosylation level is increased in SCC-ED.

With regard to the present invention, in order to investigate a lung cancer diagnosis marker, identification of a plurality of fucosylated serum proteins exhibiting decreased or increased expression in an SCLC patient, compared to a normal person, has been performed by: depleting albumin and IgG that hold 60% or more of each of the serum of a normal person (Healthy), the serum of an early SCLC patient (SCC-LD), and the serum of a terminal SCLC patient (SCC-ED); isolating a fucosylated glycoprotein from these serum samples, respectively; concentrating the same; isolating the concentrated glycoprotein from 1D-SDS PAGE gel; conducting pre-treatment of the same by In gel tryptic digestion to form a peptide condition; analyzing the pre-treated peptide using a mass spectrometer to identify 80 to 100 fucosylated proteins per each serum sample; and comparing and assaying the above three serum samples through Scaffold 2 software.

The term "diagnosis" used herein means identifying the presence or characteristics of a pathological condition. With regard to the purposes of the present invention, the diagnosis is to identify the onset of lung cancer.

Lung cancer has four major histological forms, and is classified into two types, i.e., SCLC and NSCLC, wherein NSCLC includes lung adenocarcinoma, squamous cell carcinoma and large cell lung carcinoma. However, the purpose of present invention is for diagnosing SCLC among the histological forms of lung cancer.

The antibody used in the present invention may include a whole type antibody (hereinafter, 'whole antibody') or a functional fragment thereof. The whole antibody may be in a polymer form containing monomers, or at least two whole antibodies bonded together. The functional fragment of the antibody may comprise an antibody having a heavy chain or light chain variable region of the whole antibody, which substantially recognizes the same epitope as that recognized by the whole antibody.

The functional fragment of the antibody described above may include, but is not limited to, single chain variable region fragments such as (scFv), (scFv)2, Fab, Fab' and F(ab')2, etc. Such a single chain variable region (scFv) refers to an antibody fragment wherein a heavy chain variable region is linked to a light chain variable region through a linker peptide to form a single chain polypeptide.

The antibody may be bound to different molecules such as fluorescent materials, radioactive materials and/or proteins, thus being modified. The modified antibody may also be formed by chemically modifying an antibody. The above modification methods are generally used in the art. In addition, the above antibody may comprise a chimeric antibody formed by combining a non-human antibody-derived variable region with a human antibody-derived constant region or otherwise, a humanized antibody formed by combining a human antibody-derived framework region (FR) that contains a complementarity determining region derived from a non-human antibody, with a constant region derived from a human antibody. The antibodies described above may be prepared by any conventional method well known in the art.

The diagnostic composition of the present disclosure may further include any reagent known in the art and generally used for immunological assay in addition to an antibody specific to a protein. The immunological assay used herein may include any method capable of measuring antigen-antibody binding.

Also disclosed is a diagnostic kit for SCLC, including the above composition as an effective ingredient.

The diagnostic kit may include, for example, a lateral flow assay kit based on immune-chromatography in order to detect a specific protein in a sample. The lateral flow assay kit may consist of: a sample pad receiving a sample thereon; a releasing pad coated with a detecting antibody; a developing membrane (for example nitrocellulose) or strip wherein the sample moves, is isolated, and undergoes antigen-antibody reaction; and an absorption pad.

Also disclosed is a micro-array for diagnosis of SCLC, including the above composition as an effective ingredient.

The micro-array is generally used to attach an antibody on the surface of a slide glass treated with a specific reagent, in order to detect a protein specifically adhered to the antibody by an antigen-antibody reaction.

Alternatively, the present invention provides a diagnostic composition for lung cancer, which includes a primer or probe specific to a nucleic acid encoding serum paraoxonase 1.

According to the present invention, the above protein is characterized by fucosylation and shows decreased expression in the serum of an SCLC patient, compared to the serum of a normal person.

According to the present invention, the serum paraoxonase 1 protein shows decreased expression in SCC-ED, compared to HE. However, as compared to HE, the fucosylation level is increased in SCC-ED.

According to the present invention, the above protein is characterized by fucosylation and shows increased expression in the serum of an SCLC patient, compared to the serum of a normal person.

Detection of a specific nucleic acid using the primer described above may be performed by amplifying a sequence of a target gene using an amplification method such as PCR and identifying such amplification of the gene according to a conventional method known in the art. Also, detection of a specific nucleic acid using the probe described above may be performed by contacting the probe with a sample nucleic acid under desired conditions and identifying the existence of hybridized nucleic acid.

Herein, the "primer" refers to a nucleic acid sequence having a short free hydroxyl group and substantially means a short nucleic acid sequence capable of forming a base pair with a complementary template and functioning as a starting point for copying a template strand.

The "probe" used herein refers to a nucleic acid fragment such as RNA or DNA, consisting of several to several hundred bases that can be specifically bound to mRNA, and may be labeled to identify the existence of a specific mRNA. The probe may be prepared in the form of an oligonucleotide probe, single chain DNA probe, double chain DNA probe, or RNA probe, etc., and be labeled by biotin, FITC, rhodamine, DIG, etc. or otherwise, by radioactive isotopes.

In addition, the probe may be labeled by a detectable material, for example, a radioactive label providing a desired signal and having a sufficient half-life. The labeled probe may be hybridized to a nucleic acid on a solid support, as is known in the art.

A method of detecting a specific nucleic acid using the probe or primer described above may include, but is not limited to, polymerase chain reaction (PCR), DNA sequencing, RT-PCR, primer extension, oligonucleotide extension assay, allele specific PCR, RNase mismatch cleavage, single strand conformation polymorphism, SCCP and heteroduplex simultaneous assay, denaturing gradient gel electrophoresis (DGGE), denaturing high-pressure liquid chromatography (MHPLC), hybridization, DNA chip, or the like.

The diagnostic composition for lung cancer here disclosed may further include a reagent generally used for detection of the nucleic acid described above. For instance, deoxynucleotide triphosphate (dNTP), a heat-resistant polymerase or a metal ion salt such as magnesium chloride, which are required for PCR, may be included. Alternatively, dNTP or sequenase required for sequencing may be included.

Preferably, the diagnostic composition for lung cancer may be provided in the form of a diagnostic kit or micro-array. For instance, a RT-PCR kit including each primer pair specific to at least one gene selected from a group consisting of the proteins described in the present invention, a DNA chip including a substrate that has cDNA of a marker gene or oligonucleotide attached thereto according to the present invention, and so forth, may be provided but is not be limited thereto.

Further, the present invention provides a method of detecting serum paraoxonase 1 as a lung cancer marker, from a biological sample, through antigen-antibody binding reaction that uses an antibody specifically bound to at least one protein selected from the a lung cancer marker, from a biological sample, through antigen-antibody binding reaction that uses an antibody that specifically binds to at least one protein selected from the above group.

According to the present invention, it is possible to diagnose or predict at an early stage a prognosis of lung cancer through antigen-antibody binding reaction that uses an antibody that specifically binds the protein above, as a lung cancer marker, to detect the above protein from a biological sample. More particularly, after fractionating any of these proteins through electrophoresis in SDS-PAGE, then transferring and fixing the fractionated protein to a fixture, an antibody that specifically binds to the above protein is added thereto to perform antigen-antibody binding, and then, an expression level of the protein is measured. In other words, as a result of measuring and comparing protein expression levels in tissues of both a lung cancer patient and a normal person, if the expression level of the protein in the tissue of the lung cancer patient is higher than that in the tissue of the normal person, the existence of lung cancer may be diagnosed or the possibility of lung cancer may be predicted.

According to the present invention, the lung cancer is preferably SCLC.

The biological sample used herein may include, but is not limited to, samples such as tissue, cell, whole blood, serum, plasma, saliva, sputum, and urine, which are obtained from mammals. Such samples may be preliminarily treated by filtration, distillation, extraction, concentration, deactivation of obstructive components, addition of a reagent, and so forth, before they are used for detection.

As a fixture for antigen-antibody binding reaction, a nitrocellulose membrane, a polyvinylidene difluoride membrane, a 96-well plate consisting of polyvinyl resin or polystyrene resin, a slide glass made of glass, or the like, may be used.

The antigen-antibody binding may be performed by any conventional method, for example, enzyme-linked test, antigen-antibody aggregation, and so forth.

Hereinafter, preferred embodiments are provided to more clearly understand the present invention. However, the following examples are only given for more concretely describing the present invention and may not be construed as limitation of the scope of the present invention.

### EXAMPLE

### Example 1: Blood sample collection and depletion of large quantities of proteins from (blood) serum

Blood samples were collected from 29 normal persons, 25 early SCLC patients and 29 terminal SCLC patients at Samsung Hospital. Each of the blood samples was centrifuged at 4°C and 12,000 rpm for 10 minutes to obtain a serum and the serum was stored at -70°C until it was used for the experiment.

For mass spectroscopy of all serums obtained above, that is, the serums of normal persons (Healthy; 29 persons), the serums of early SCLC patients (SCC-LD; 25 persons), and the serums of terminal SCLC patients (SCC-ED; 29 persons), 5 persons were selected from each serum sample group and subjected to the experiment. Albumin and IgG holding 60% or more of the serum in each serum sample group were depleted using an Agilent Technologies (Multi Affinity Removal Spin Cartridge) depletion kit.

### Example 2: Isolation and concentration of fucosylated glycoprotein from serum sample

Agarose-conjugated AAL (Aleuria aurantia lectin) was placed in a spin column and, after diluting a serum sample free from albumin and IgG in 500 *µ*ℓ buffered solution, the diluent was loaded into the spin column to isolate an elution and a flow-through fraction. The isolated elution was concentrated to provide a fucosylated glycoprotein. In order to verify the isolation of the fucosylated glycoprotein, the fucosylated glycoprotein was detected by AAL lectin blotting. The lectin blotting is substantially similar to immune-blotting, however, uses biotinylated AAL and HRP-conjugated streptavidin instead of primary and secondary antibodies, respectively.

### Example 3: SDS-PAGE separation and Coomassie blue staining of the isolated fucosylated glycoprotein

The elution (concentrated fucosylated glycoprotein) and the flow-through fraction were respectively treated by electrophoresis using an acrylamide gel and the gel was washed three times with distilled water for 5 minutes. Then, using a bio-safe Coomassie staining solution (Coomassie G250 stain: Bio-Rad), the gel was stained over 1 hour while softly agitating at room temperature. Next, the gel was decolorized in distilled water overnight then washed with distilled water, thus completing the staining process.

FIG. 1(A) illustrates results of SDS PAGE gel electrophoresis of serum samples obtained by depleting albumin and IgG from three serum sample groups of the present invention (i.e., Healthy (HE), SCC-LD (LD) and SCC-ED (ED)) (T: crude serum, B: albumin and IgG as proteins adhered to a depletion column, D: albumin and IgG-depleted serum). As shown in FIG. 1(A), it was found that albumin and IgG were depleted from all of the three serum sample groups.

FIG. 1(B) illustrates results of observing the fucosylated glycoprotein through AAL lectin blotting (T: crude serum, Flow through: sample not adhered to the fucosylation enriched column, elution: fucosylated glycoprotein adhered to the fucosylation column). As a result of observing the elution containing the fucosylated glycoprotein isolated from the albumin and IgG-depleted serum through AAL lectin blotting, it was found that the elution sample adhered to the lectin column showed strong expression of the fucosylated glycoprotein.

FIG. 1(C) illustrates Coomassie blue staining results of both the sample not adhered to the fucosylation column and the sample adhered to the same (Flow through: sample not adhered to the fucosylation column, elution: sample adhered to the fucosylation column).

### Example 4: Identification of fucosylated glycoprotein

After separating the concentrated fucosylated glycoprotein obtained in Example 2 from 1D-SDS PAGE gel, the separated product was pre-treated to form a peptide condition through in-gel tryptic digestion. The pre-treated three serum sample groups (i.e., Healthy (HE), SCC-LD (LD) and SCC-ED (ED)) were analyzed using a mass spectrometer, thus performing identification of proteins. Next, comparing and assaying these three serum sample groups using Scaffold 2 software, proteins specific to SCLC were identified.

Mass spectroscopic results of fucosylated glycoproteins specific to SCLC, which were the fucosylated glycoproteins concentrated in the three serum sample groups of the present invention, respectively (i.e., Healthy (HE), SCC-LD (LD) and SCC-ED (ED)), are shown in FIG. 2. Comparison and assay were repeated twice and numeral values on the 'y' axis represented on the graph indicate the number peptides measured by the mass spectrometer.

As shown in FIG. 2, as the mass spectroscopic results of the fucosylated glycoproteins specific to SCLC, which were the fucosylated glycoproteins concentrated in the three serum sample groups, respectively (i.e., Healthy (HE), SCC-LD (LD), and SCC-ED (ED)), it was found that the serum paraoxonase 1 (PON1) protein was decreased in SCC-ED. Accordingly, the serum paraoxonase 1 (PON1) protein was identified as the glycoprotein specific to SCLC.

**[TABLE 1]**

| Identified protein | IPI number | Number of Assigned Spectra | | | Coverage (%) | | | Fold | |
|---|---|---|---|---|---|---|---|---|---|
| | | Health y (HE) | SCC-LD (LD) | SCC-ED (ED) | Healthy (HE) | SCC-LD (LD) | SCC-ED (ED) | HE/LD fold | HE/ED fold |
| Isoform 1 of Lipopolysa ccharide-responsive and beige-like anchor protein | IPI00002255 | 5 | 0 | 0 | 0 | 0 | 0 | - | - |
| | | 4 | 0 | 3 | 1 | 0 | 0.7 | - | 1.33 |
| Apolipoprotein D | IPI00006662 | 5 | 3 | 0 | 24 | 13 | 0 | 2 | - |
| | | 4 | 0 | 0 | 13 | 0 | 0 | - | - |
| Serotransferrin | IPI00022463 | 38 | 22 | 19 | 28 | 18 | 17 | 2 | 2.32 |
| | | 42 | 18 | 26 | 33 | 27 | 25 | 2.35 | 1.6 |
| Kallistatin | IPI00328609 | 4 | 0 | 0 | 4 | 0 | 0 | - | - |
| | | 5 | 0 | 0 | 13 | 0 | 0 | - | - |
| Plasma kallikrein | IPI00654888 | 10 | 5 | 5 | 8 | 5.3 | 7.8 | 2.4 | 2.4 |
| | | 9 | 0 | 6 | 11 | 0 | 8 | - | 1.5 |
| Lumican | IPI00020986 | 9 | 12 | 5 | 12 | 16 | 8.6 | 0.92 | 2.2 |
| | | 14 | 7 | 5 | 17 | 9.5 | 12 | 1.86 | 2.6 |
| Histidine-rich glycoprote in | IPI00022371 | 30 | 26 | 22 | 15 | 11 | 13 | 1.35 | 1.59 |
| | | 33 | 24 | 17 | 15 | 13 | 13 | 1.41 | 1.82 |
| Alpha-2-HS-glycoprotein | IPI00022431 | 3 | 5 | 0 | 4 | 3.9 | 0 | 0.8 | - |
| | | 5 | 6 | 2 | 4 | 3.9 | 3.9 | 0.83 | 2.5 |
| Alpha-1B-glycoprotein | IPI00022895 | 10 | 9 | 0 | 14 | 9.7 | 0 | 1.33 | - |
| | | 12 | 0 | 5 | 10 | 0 | 7.1 | - | 2.2 |
| Apolipoprotein A-IV | IPI00304273 | 13 | 13 | 5 | 21 | 20 | 7.8 | 1.15 | 3 |
| | | 21 | 9 | 6 | 33 | 17 | 11 | 2.5 | 3.33 |

**[TABLE 2]**

| Identified protein | IPI number | Number of Assigned Spectra | | | Coverage (%) | | | Fold | |
|---|---|---|---|---|---|---|---|---|---|
| | | Healthy (HE) | SCC-LD (LD ) | SCC-ED (ED) | Healthy (HE) | SCC-LD (LD) | SCC-ED (ED) | LD/HE fold | ED/HE fold |
| Haptoglobin | IPI00641737 | 135 | 340 | 629 | 32 | 38.0 | 41.0 | 2.18 | 3.97 |
| | | 139 | 353 | 636 | 30 | 38.0 | 41.0 | 2.52 | 4.74 |
| Hemopexin | IPI00022488 | 7 | 13 | 15 | 7 | 11.0 | 11.0 | 1.63 | 1.88 |
| | | 11 | 17 | 20 | 7 | 11.0 | 12.0 | 1.60 | 2.00 |
| C4b-binding protein alpha chain | IPI00021727 | 0 | 4 | 7 | 0 | 5.2 | 7.7 | - | - |
| | | 0 | 4 | 5 | 0 | 7.7 | 4.5 | - | - |
| Complement component C9 | IPI00022395 | 0 | 2 | 2 | 0 | 2.0 | 4.5 | - | - |
| | | 0 | 2 | 3 | 0 | 3.8 | 2.0 | - | - |
| CD5 antigen-like protein | IPI00025204 | 6 | 13 | 9 | 12 | 19.0 | 15 | 1.86 | 1.29 |
| | | 7 | 14 | 6 | 11 | 18.0 | 11 | 1.86 | 0.86 |
| Inter-alpha-trypsin inhibitor heavy chain H2 | IPI00305461 | 0 | 3 | 0 | 0 | 2.7 | 0 | - | - |
| | | 0 | 5 | 0 | 0 | 2.5 | 0 | - | - |
| Serum amyloid P component | IPI00022391 | 0 | 2 | 6 | 0 | 9 | 14.0 | - | - |
| | | 0 | 0 | 5 | 0 | 0 | 19.0 | - | - |
| Isoform 2 of Inter-alpha-trypsin inhibitor heavy chain H4 | IPI00218192 | 13 | 13 | 26 | 6 | 5.6 | 7.0 | 0.87 | 1.73 |
| | | 8 | 28 | 27 | 3 | 6 | 10.0 | 3.25 | 3.25 |
| Isoform 1 of Haptoglobin -related protein | IPI00477597 | 0 | 0 | 10 | 0 | 0 | 6.9 | - | - |
| | | 0 | 0 | 6 | 0 | 0 | 8.3 | - | - |
| ALPHA-1-antichymotrypsin | IPI00550991 | 19 | 30 | 38 | 12 | 17 | 18.0 | 1.36 | 1.73 |
| | | 23 | 31 | 42 | 17 | 18 | 18.0 | 1.32 | 1.86 |
| Isoform 1 of Alpha-1-antitrypsin | IPI00553177 | 13 | 24 | 29 | 19 | 20 | 22.0 | 1.26 | 1.53 |
| | | 15 | 31 | 27 | 15 | 23 | 20.0 | 2.07 | 1.86 |

In addition, it was demonstrated from analysis of the fucosylated glycoprotein that the glycoprotein was also decreased in the serum of the SCLC patient, in addition to the serum paraoxonase 1 (PON1), compared to the serum of the normal person. This result is shown in Table 1. Alternatively, glycoproteins increased in the serum of the SCLC patient, compared to the serum of the normal person, were identified and these results are shown in Table 2.

### Example 5: Assay of fucosylation level of serum paraoxonase 1 protein

In order to identify whether the serum paraoxonase 1 (PON1) protein as a protein showing a decrease pattern in the terminal SCLC patient has a sugar chain containing fucose, the following experiments have been performed. More particularly, the serum paraoxonase 1 (PON1) protein identified in Example 4 was treated with PNGase F as a sugar chain removing enzyme to inspect whether the sugar chain containing fucose is bonded to the serum paraoxonase I (PON1) protein. If a mass of the protein is reduced when the sugar chain was depleted from the protein by treating the serum paraoxonase 1 (PON1) with PNGase F as a sugar chain removing enzyme, it can be identified that the sugar chain was bonded to the protein.

As shown in FIG. 3, when the serum paraoxonase 1 (PON1) protein was treated with PNGase F as a sugar chain removing enzyme, it was found that a band at the mass of 40 kDa was decreased to the mass of about 37 kDa. Accordingly, it can be identified that a sugar chain containing fucose was bonded to the serum paraoxonase 1 (PON1) protein as a protein specific to SCLC (Crude - crude serum, Untreated - sample not treated using PNGase F enzyme, Treated - sample treated using PNGase F enzyme).

### Example 6: Western blotting and densitometry assay of serum paraoxonase 1 protein

In order to verify expression phases of the serum paraoxonase 1 (PON1) protein, increased numbers of serum sample groups (i.e., serums of normal persons (Healthy, 29 persons), serums of early SCLC patients (SCC-LD, 25 persons), and serums of terminal SCLC patients (SCC-ED, 29 persons)) were subjected to a western blotting test. Also, results of the western blotting test were subjected to densitometry. Alternatively, a control group used the same serum sample in each blot.

As shown in FIG. 4, it was identified that the serum paraoxonase 1 (PON1) protein had expression levels more considerably decreased in the serums of early SCLC patients (SCC-LD) and terminal SCLC patients (SCC-ED) in this order, rather than the serums of normal persons.

### Example 7: Measurement of fucosylation level of serum paraoxonase 1 protein

A level of change in fucosylation of the serum paraoxonase 1 (PON1) protein in each of the serum sample groups (i.e., serums of normal persons (Healthy; HE, 29 persons), serums of early SCLC patients (SCC-LD; LD, 25 persons), and serums of terminal SCLC patients (SCC-ED; ED, 29 persons)) was determined by a hybrid ELISA method. The hybrid ELISA method is substantially similar to a sandwich ELISA method, however, uses a coating of a specific protein antibody and also uses, after a sample reaction, biotinylated AAL and HRP-conjugated streptavidin instead of primary and secondary antibodies, respectively. The experiment was repeated twice.

As shown in FIG. 5, an expression level of the fucosylated serum paraoxonase 1 (PON1) protein was more greatly increased as SCLC progressed to a terminal stage, compared to a normal person. That is, this means that the binding of a fucose sugar to serum paraoxonase 1 (PON1) protein is more increased as SCLC progresses to a terminal stage.

### Example 8: Determination of sensitivity and specificity of serum paraoxonase 1 protein level and fucosylation level

FIG. 6 illustrates levels of serum paraoxonase 1 proteins through western blotting and levels of fucosylated serum paraoxonase 1 by hybrid lectin ELISA through ROC curve, with regard to three serum sample groups (i.e., serums of normal persons (Healthy; HE, 29 persons), serums of early SCLC patients (SCC-LD; LD, 25 persons), and serums of terminal SCLC patients (SCC-ED; ED, 29 persons)), respectively.

As a result, it was demonstrated that an area under curve (AUC) at a level of the fucosylated serum paraoxonase 1 (PON1) is higher than AUC at a level of the serum paraoxonase 1 (PON1) protein measured by western blotting.

The above method was firstly disclosed in the present invention and suggests that measuring an increase in fucosylation level of serum paraoxonase 1 (PON1) protein rather than measuring a decrease in protein mass of the serum paraoxonase 1 (PON1) protein may exhibit a higher accurate diagnosis rate.

## Claims

1. A method for diagnosis of small cell lung cancer from a biological sample by measuring the fucosylation level of serum paraoxanase 1 (PON1) protein.

2. A method according to claim 1 using a composition comprising an antibody that specifically binds to serum paraoxonase 1 (PON1) protein.

3. A method according to anyone of the previous claims, wherein the protein is fucosylated.

4. A method according to anyone of the previous claims, wherein the protein shows decreased expression in a serum of a small cell lung cancer patient, compared to a serum of a normal person.

5. A method according to anyone of the previous claims, wherein the protein shows decreased expression in a serum of a small cell lung cancer patient in a terminal stage, compared to a serum of a normal person.

6. A method according to anyone of the previous claims, wherein the protein shows an increased fucosylation level in a serum of a small cell lung cancer patient in a terminal stage, compared to a serum of a normal person.

## Patentansprüche

1. Verfahren zur Diagnose von kleinzelligem Lungenkrebs aus einer biologischen Probe durch Messung des Fukosylierungs-Levels des Serum Paraoxanase 1 (PON1) Proteins.

2. Verfahren nach Anspruch 1 unter Verwendung einer Zusammensetzung umfassend einen Antikörper, der spezifisch an das Serum Paraoxonase 1 (PDN1) Protein gebunden ist

3. Verfahren nach einem der vorhergehenden Ansprüche, wobei das Protein fukosyliert ist.

4. Verfahren nach einem der vorhergehenden Ansprüche, wobei das Protein eine verringerte Expression in einem Serum eines kleinzelligen Lungenkrebs-Patienten im Vergleich zu einem Serum einer normalen Person zeigt.

5. Verfahren nach einem der vorhergehenden Ansprüche, wobei das Protein eine verringerte Expression in einem Serum eines kleinzelligen Lungenkrebs Patient in einem Endstadium im Vergleich zu einem Serum einer normalen Person zeigt.

6. Verfahren nach einem der vorhergehenden Ansprüche, wobei das Protein ein erhöhtes Fucosylierungs-Level in einem Serum eines kleinzelligen Lungenkrebs-Patienten in einem Endstadium im Vergleich zu einem Serum einer normalen Person zeigt.

## Revendications

1. Procédé pour le diagnostic d'un cancer du poumon à petites cellules dans un échantillon biologique en mesurant les niveaux de règles fucosylation de la protéine sérum paraoxanase-1 (PON1).

2. Procédé selon la revendication 1 en utilisant une composition comprenant un anticorps qui se lie spécifiquement au de la protéine sérum paraoxanase-1.

3. Procédé selon l'une quelconque des revendications précédentes, dans lequel ladite protéine est fucolysée.

4. Procédé selon l'une quelconque des revendications précédentes, dans lequel ladite protéine montre une diminution de l'expression dans un sérum d'un patient atteint d'un cancer du poumon à petites cellules par rapport à un sérum d'une personne normale.

5. Procédé selon l'une quelconque des revendications précédentes, dans lequel ladite protéine montre une diminution de l'expression dans un sérum d'un patient atteint de petit cancer du poumon à petites cellules à un stade final par rapport à un sérum d'une personne normale.

6. Procédé selon l'une quelconque des revendications précédentes, dans lequel ladite protéine montre un niveau fucosylation augmenté dans le sérum d'un petit patients atteint de petit cancer du poumon à petites cellules dans une stade finale en comparaison avec un sérum d'une personne normale.
